(19) Europäisches Patentamt
European Patent Office
Office européen des brevets

(11) Numéro de publication : **0 307 277 B1**

# (12) FASCICULE DE BREVET EUROPEEN

(45) Date de publication du fascicule du brevet : **24.07.91 Bulletin 91/30**

(51) Int. Cl.$^5$ : **A61K 35/78,** A61K 35/56, // (A61K35/78, 35:56, 31:80, 31:695)

(21) Numéro de dépôt : **88402164.3**

(22) Date de dépôt : **26.08.88**

## (54) Produit à base de silanol pour les soins des vaisseaux lymphatiques superficiels.

(30) Priorité : **09.09.87 FR 8712480**

(43) Date de publication de la demande : **15.03.89 Bulletin 89/11**

(45) Mention de la délivrance du brevet : **24.07.91 Bulletin 91/30**

(84) Etats contractants désignés : **BE DE GB IT LU NL**

(56) Documents cités :
FR-A- 1 234 213
FR-A- 1 332 198
US-A- 4 393 045

(73) Titulaire : **Seguin, Marie-Christine
Périgord 1 6 Lacets Saint-Léon
Monte-Carlo (MC)**
Titulaire : **Gueyne, Jean
Périgord 1 6 Lacets Saint Léon
Monte-Carlo (MC)**

(72) Inventeur : **Seguin, Marie-Christine
Périgord 1 6 Lacets Saint-Léon
Monte-Carlo (MC)**
Inventeur : **Gueyne, Jean
Périgord 1 6 Lacets Saint Léon
Monte-Carlo (MC)**

(74) Mandataire : **Bertrand, Didier et al
c/o S.A. FEDIT-LORIOT & AUTRES CONSEILS EN PROPRIETE INDUSTRIELLE 38, Avenue Hoche
F-75008 Paris (FR)**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).



Jouve, 18, rue Saint-Denis, 75001 PARIS

## Description

La présente invention concerne un produit pour les soins des vaisseaux lymphatiques superficiels. Elle comprend notamment un gel ou une émulsion destiné aux massages des parties du corps, dont on veut soigner les vaisseaux lymphatiques.

L'importance du système lymphatique est souvent trop négligée,et la pharmacopée est pauvre en compositions susceptibles d'améliorer l'état des vaisseaux lymphatiques. Et pourtant ces vaisseaux sont sujets à diverses atteintes et affections qui demandent à être soignés, sous peine de complications dermatologiques ou même générales de l'individu. Ainsi, devrait-on combattre les altérations de la tunique lymphatique et la baisse de sa résistance, les obstructions des capillaires, l'épaississement de la lymphe, la formation de malonaldéhyde cytotoxique, et d'autres anomalies. Or, la pharmacopée actuelle, dans sa partie dermatologique, s'occupe presqu'uniquement des veines et des capillaires sanguins, sans mettre à disposition des médicaments spécifiques du système lymphatique. Cette situation est sans inconvénient, lorsque les produits, proposés contre les troubles du fonctionnement veineux, sont également efficaces vis-à-vis des canaux lymphatiques ; ainsi, par exemple, les crèmes ou pommades à base d'aescine, d'extrait de mélilot ou/et d'hétérosides stéroliques de ruscus aculeatus, conviennent-elles en général tant dans le cas de l'insuffisance veineuse que dans celle des vaisseaux lymphatiques ; mais il n'en est plus de même pour les nombreux autres médicaments connus.

D'autre part, on ne connaît pas de produits capables, à la fois, de régénérer la tunique lymphatique, de régler la viscosité de la lymphe et d'exercer une action anti-oedémateuse. Il est connu que l'engorgement des canaux lymphatiques, dont la fonction est l'élimination de grosses molécules, toxines, bactéries, lipides, peptides, etc., entraîne un oedème des tissus voisins et une certaine cytotoxicité. Pour palier ces inconvénients, on a utilisé différents moyens physiques, notamment le drainage lymphatique manuel, ou la pressothérapie séquentielle ; cependant, à défaut d'un agent biochimique, susceptible d'être employé conjointement avec ces procédés, les résultats laissent à désirer.

La présente invention comble cette lacune de l'art antérieur : elle apporte une composition destinée spécifiquement aux soins des vaisseaux lymphatiques, capable d'agir simultanément sur plusieurs des atteintes mentionnées plus haut. Ainsi, indépendamment de l'état veineux du patient, donc contrairement à ce qui était pratiqué jusqu'à présent, le produit nouveau, suivant l'invention, peut améliorer la texture de la tunique lymphatique, et augmenter la résistance des capillaires correspondants, tout en ramenant la viscosité de la lymphe à un niveau approprié, et en agissant sur les microsphincters, pour les relaxer, ce qui produit une action anti-oedémateuse.

Ces nouveaux résultats, importants, sont obtenus suivant l'invention par l'association de certains produits actifs naturels avec un ou plusieurs silanols qui, à côté de leur action spécifique, propre, renforcent celle des produits naturels ou bien même induisent à ceux-ci des propriétés nouvelles.

Suivant un premier trait de l'invention, la nouvelle composition, pour les soins des vaisseaux lymphatiques, est caractérisée en ce qu'elle contient un ou plusieurs silanols ou dérivés de silanol accompagnés d'acide concholytique. Pour les applications cutanées, cette composition est mise sous la forme de gel, d'émulsion ou de pommade, aqueuses, selon la technique bien connue qui — par conséquent — n'a pas à être décrite ici.

Les teneurs préférées en silanols, ou/et en leurs dérivés, sont d'environ 0,05 à 0,5% en poids, les proportions d'acide concholytique étant également de 0,05 à 0,5%.

Les silanols, convenant particulièrement à la réalisation de l'invention, sont du type $R_n\text{-}Si(OH)_{(4-n)}$ où R est un radical hydrocarboné, surtout alkyle en $C_1$ à $C_{18}$, n est 1 à 3, tous ou partie des H pouvant être remplacés par un alkyle en $C_1$ à $C_{18}$. Dans les silanols les plus courants R est un alkyle en $C_1$ à $C_4$.

On a désigné plus haut, par le terme "dérivés de silanol", les composés dans lesquels un silanol est combiné à une autre molécule, en particulier à un acide. De tels dérivés, connus dans l'art, ont été décrits, par exemple, dans le brevet français n° 1 234 213 qui en donne quelques formules à la page 2. Dans ces composés

$$R_nSi(OH)_{(4-n)}.X.R'COOH, \quad (1)$$

R et n ont la même signification que plus haut, X est en général le nombre des OH attachés à Si, soit (4-n) lorsqu'aucun des H de ces oxydriles n'est remplacé par un groupe hydrocarboné. En tant qu'acide R'COOH on peut avoir différents acides organiques, pharmaceutiquement acceptables, dont des amino-acides. A titre non limitatif, on peut citer les acides acétique, sébacique, citrique, tartrique, lactique, nicotinique, ascorbique, folique, salicylique, acétyl-salicylique, lactique, hyaluronique, aspartique, glutamique, glycine, sérine, thréonine, tyrosine, etc.

En tant que dérivés de silanol peuvent également être employés des siloxanes, par exemple du type

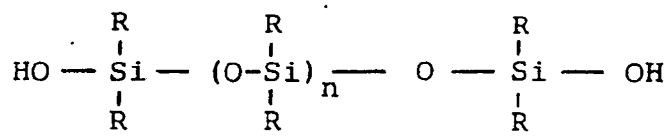

où R est un groupe hydrocarboné et n un nombre de 0 à 20, mais de préférence 0 à 4.

En ce qui concerne l'acide concholytique, sa source particulièrement pratique étant constituée par des coquilles de mollusques, et surtout par de la nacre, il est avantageusement pris sous la forme d'un extrait de nacre. Cet extrait aqueux résulte de l'hydrolyse acide, ménagée, de la nacre.

Dans une forme d'exécution particulière de l'invention, la composition contient également un ou plusieurs "protéosilanes", c'est-à-dire des combinaisons de composés organiques du silicium avec des protides. L'action conjointe des silanols et protéosilanes est alors renforcée et localisée sur les lymphatiques superficiels. Les protéosilanes peuvent être représentés schématiquement par la formule

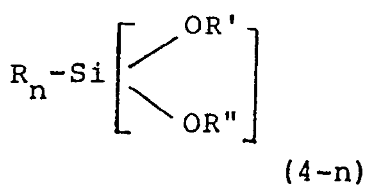 ...... (3)

où R est un groupement organique, en particulier alkyle ou alcényle en $C_1$ à $C_{18}$, n = 1 à 3, R' un groupe actif vis-à-vis de la peau, par exemple théophylline, acide théophylline acétique, glycyrrhizique, acétyl-tyrosine, etc., et R" un groupe protidique, lipoprotidique ou nucléoprotidique, en particulier peptide, polypeptide, ou cyclopeptide, notamment albumine, fibrine, collagène, kératine, élastine ou autre groupe protéinique.

Pour les compositions suivant l'invention conviennent particulièrement bien les dérivés du silicium selon la formule (3), dans lesquels R est $CH_3$, n = 1 ou 2, R' un des groupes acides susmentionnés et R" l'élastine solubilisée par hydrolyse partielle.

Suivant un autre trait de l'invention, la composition contient de préférence une huile essentielle, notamment huile terpénique, par exemple l'huile de lavande. De telles huiles modifient la viscosité et la qualité de la lymphe, grâce à la présence de silanols qui favorisent leur pénétration et incorporation au liquide lymphatique. La proportion de l'huile est de préférence de 0,05 à 2% en poids.

Le rôle de glycosides, notamment de rutosides ou saponosides, étant connu pour l'augmentation de la résistance des capillaires, ces substances, lorsqu'elles sont présentes dans la composition suivant l'invention, bénéficient d'une activation par le silanol de leur action normalisatrice de texture de la tunique lymphatique. De telles substances sont, par exemple, des polyphénols dans les cas des extraits de Piloselle, de Centella asiatica et d'Arnica, cette normalisation entraînant une réduction des oedèmes ; ou bien ce sont des hétérosides stéroliques pour l'extrait de Ruscus, avec — comme effet second — l'augmentation de la résistance capillaire lymphatique. Aussi, dans une variante de l'invention, la composition renferme un ou plusieurs glycosides de ce type, par exemple de la rutine, de l'extrait de ruscus, de l'hydroxyéthyl rutoside ou similaire. Des adjuvants favorables à la normalisation de texture de la tunique lymphatique sont les extraits de piloselle, de centella asiatica ou/et d'arnica.

L'action relaxante sur les microsphincters, qui s'accompagne d'une action anti-oedémateuse de la composition suivant l'invention, peut être renforcée par l'adjonction d'extraits naturels tels que par exemple ceux de ginkgo ou/et de mélilot. Les proportions des extraits naturels, dans la composition, varient de préférence entre environ 0,05 et 2% en poids.

Un gel ou une émulsion, contenant du silanol et de l'acide concholytique, appliqué topiquement, présente l'intéressant avantage d'exercer une action localisée sur les vaisseaux lymphatiques superficiels, en normalisant la texture de leur tunique, réglant la viscosité de la lymphe, tout en exerçant une action anti-oedémateuse. Les effets sont bien plus sensibles que lorsqu'on applique un seul des deux constituants en question. L'action peut être bien plus intense et plus complète lorsqu'on adjoint à ces constituants certains extraits de plantes, des protéosilanes et des dérivés terpéniques. La régénération des fibres de collagène et d'élastine, et la restructuration des protéoglucanes, dues au silanol, sont accompagnées d'une intervention dans le métabolisme des corps gras ; ainsi, lors de la lipolyse, le silanol favorise-t'il la formation d'acides gras insaturés et les protège de la péroxydation cytotoxique, susceptible d'élever la viscosité de la lymphe. D'autre part, la réduction des oedèmes par modification de la perméabilité capillaire, sous l'effet du silanol, est fortement potentialisée par

l'acide concholytique.

L'invention est illustrée par les exemples non limitatifs qui suivent.

EXEMPLE 1

Dans un gel, formé par 100 ml d'eau, renfermant 1 g de résine hydrosoluble de carboxypolyméthylène connue sous la dénomination de "CARBOPOL", on a dispersé 0,1 g d'acide concholytique pris à l'état de son extrait de nacre, ainsi que 0,04 g de dilactate de diméthyl-silanol

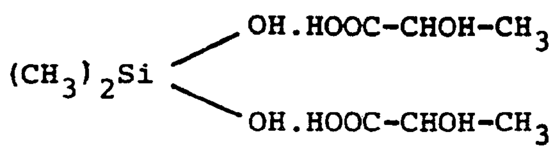

et 0,06 g d'hyaluronate de méthyl silane triol.

$$CH_3\text{-}Si(OH)_3.HOOC\text{-[groupe hyaluronique]}.$$

Ces deux dérivés de silanol ont été employés sous la forme de solutions aqueuses à 1%. En outre, 0,1 g d'huile de lavande était dispersée dans le gel.

La composition obtenue s'est révélée très efficace pour la régénération, renforcement des vaisseaux lymphatiques et diminution de la viscosité de la lymphe, surtout par son application avec massages.

EXEMPLE 2

A 100 ml de gel de l'exemple 1 on a incorporé 0,5 g de protéosilane selon la formule donnée plus haut, dans laquelle R est $CH_3$, n = 1, R' est le groupe d'acide théophylline acétique et R" celui d'élastine solubilisée. L'application de la composition donne les mêmes bons résultats que celle de l'exemple 1 avec, en outre, une amélioration localisée sur les canaux lymphatiques superficiels.

EXEMPLE 3

Le gel de l'exemple 2 fut additionné de 1 g d'extrait de ruscus qui a contribué à l'augmentation de la résistance des capillaires lymphatiques.

EXEMPLE 4

On a dispersé 1 g d'extrait de mélilot dans 100 ml du gel de l'exemple 3, pour assurer le bon écoulement du flux lymphatique, par son action relaxante sur les microsphincters.

EXEMPLE 5

Dans 100 ml de gel suivant l'exemple 3, on a dispersé 2 g d'extrait de ginkgo, ce qui a parfait le réglage du débit de lymphe, avec réduction de l'oedème.

EXEMPLE 6

Le gel selon exemple 3 a été additionné de 1 g d'extrait de piloselle, pour mieux régler le flux de la lymphe.

EXEMPLE 7

Le gel de l'exemple 4 fut additionné d 0,1 g d'hydrumine d'arnica, pour 100 ml de gel, dont l'action favorable sur la tunique lymphatique fut potentialisée par la présence de silanol.

EXEMPLE 8

Au gel de l'exemple 4 on a ajouté, pour 100 ml, 0,1 g d'hydrumine de centelle asiatique ; l'effet est similaire

à celui de l'arnica dans l'exemple 7.

EXEMPLE 9

Dans l'exemple 1 l'huile de lavande est remplacée par de l'huile de térébenthine.

EXEMPLE 10

Dans 100 ml de gel aqueux, formé de 100 ml d'eau tenant en solution 2 g de polyvinyl-pyrrolidone, on disperse les quantités en g suivantes de constituants :

| | | | |
|---|---|---|---|
| huile de lavande ...... | 0,1 | extrait hydroglycolique d'arnica enrichi en stérols de cette plante ....... | 0,1 |
| acide concholytique ... | 0,1 | " " de centelle asiatique | 0,1 |
| lactate de méthyl silane triol .......... | 0,04 | extrait de mélilot ... | 0,1 |
| hyaluronate de méthyl silane triol .......... | 0,06 | " "ruscus .... | 0,1 |
| Protéosilane E ......... | 0,1 | " " "ginkgo .... | 0,2 |
| | | " " piloselle .... | 0,1 |

Pour cette préparation les composés du Si sont employés chacun sous la forme d'une solution aqueuse à 1%.

On a désigné par "protéosilane E" le complexe selon la formule (3), dans lequel R est $C_2H_5$, n = 2, R' est un groupe d'acide acétyl tyrosine et R" celui d'élastine solubilisée.

Appliquée avec massage, cette composition produit tous les effets favorables indiqués au début de la présente description.

EXEMPLE 11

Dans 100 ml d'eau on dissout 1 g d'agent tensioactif non ionique, vendu sous la marque de fabrique TWEEN 20 (polyoxyéthylène monolaurate de sorbitane), et avec la solution obtenue on émulsionne 3 g d'huile d'amandes douces. On disperse ensuite, dans l'émulsion ainsi formée, les mêmes constituants qu'à l'exemple 10, en les mêmes proportions, de la même manière.

D'excellents résultats sont obtenus, tout comme avec le gel de l'exemple 10.

## Revendications

1. Composition aqueuse pour les soins des vaisseaux lymphatiques superficiels, caractérisée en ce qu'elle contient un ou plusieurs silanols ou dérivés de silanols conjointement avec de l'acide concholytique.

2. Composition suivant la revendication 1, caractérisée en ce qu'elle contient 0,05 à 0,5% en poids de silanols ou de leurs dérivés, la teneur en acide concholytique étant également de 0,05 à 0,5% en poids.

3. Composition suivant la revendication 1 ou 2, caractérisée en ce que le ou les silanols employés sont du type $R_nSi(OH)_{(4-n)}$, R étant un radical hydrocarboné, en particulier alkyle en $C_1$ à $C_{18}$, et n = 1 à 3, un ou plusieurs des H pouvant être remplacés par des alkyles en $C_1$ à $C_{18}$.

4. Composition suivant la revendication 1 ou 2, caractérisée en ce que les dérivés de silanols sont du type

$$R_nSi(OH)_{(4-n)}.X.R'COOH$$

où R est un groupe hydrocarboné, en particulier alkyle en $C_1$ à $C_{18}$, n est 1 à 3, R'COOH étant un acide pharmaceutiquement acceptable et X est (4-n), une partie des H du $Si(OH)_{(4-n)}$ pouvant être remplacés par un radical hydrocarboné.

5. Composition suivant la revendication 1 ou 2, caractérisée en ce que le silanol est un siloxane du type

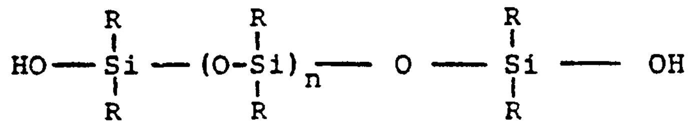

où R est un groupe hydrocarboné et n un nombre de 0 à 20, de préférence de 0 à 4.

6. Composition suivant une des revendications précédentes, caractérisée en ce qu'elle renferme un ou plusieurs protéosilanes

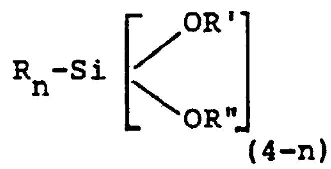

dans le(s)quel(s) R est un groupe hydrocarboné, en particulier alkyle ou alcényle $C_1$ à $C_{18}$, n est 1 à 3, R' un groupe actif vis-à-vis de la peau, notamment groupe de théophylline, d'acide théophylline acétique, d'acide glycyrrhizique ou d'acétyl-tyrosine, et R" un groupement protodique, en particulier lipoprotidique, nucléoprotidique, de peptide, de polypeptide ou de cyclopeptide, notamment d'albumine, de fibrine, de collagène, de kératine ou d'élastine, la proportion de protéosilane étant de préférence de 0,05 à 0,5% en poids.

7. Composition suivant une des revendications précédentes, caractérisée en ce qu'elle renferme une huile essentielle notamment terpénique de préférence à raison de 0,05 à 2% en poids.

8. Composition suivant une des revendications précédentes, caractérisée en ce qu'elle contient un extrait végétal, actif, en particulier d'arnica, de mélilot, ruscus, ginkgo, piloselle ou/et centella asiatica, de préférence à la proportion de 0,05 à 2% en poids.

9. Composition suivant une des revendications précédentes, caractérisée en ce qu'elle est sous la forme de gel, d'émulsion ou de pommade.

**Patentansprüche**

1. Wässrige Zusammensetzung für die Behandlung oberflächiger lymphatischer Gefäße, dadurch gekennzeichnet, daß sie ein oder mehrere Silanole oder Silanolderivate zusammen mit Conchiolinsäure (acide concholytique) enthält.

2. Zusammensetzung nach Anspruch 1, dadurch gekennzeichnet, daß sie 0,05 bis 0,5 Gew.-% Silanole oder ihre Derivate enthält und der Gehalt an Conchiolinsäure ebenfalls 0,05 bis 0,5 Gew.-% beträgt.

3. Zusammensetzung nach Anspruch 1 oder 2, dadurch gekennzeichnet, daß das oder die verwandten Silanole vom Typ $R_nSi(OH)_{(4-n)}$ sind, wobei R ein Kohlenwasserstoffrest, insbesondere $C_1$- bis $C_{18}$-Alkyl ist und n = 1 bis 3, wobei ein oder mehrere H durch $C_1$- bis $C_{18}$-Alkyl ersetzt sein können.

4. Zusammensetzung nach Anspruch 1 oder 2, dadurch gekennzeichnet, daß die Silanolderivate vom Typ $R_nSi(OH)_{(4-n)}$.X.R'COOH sind, worin R eine Kohlenwasserstoffgruppe ist, insbesondere ein $C_1$- bis $C_{18}$-Alkyl, n 1 bis 3 ist, R'COOH eine pharmazeutisch annehmbare Säure ist und X (4-n) ist, wobei ein Teil der H des $Si(OH)_{(4-n)}$ durch einen Kohlenwasserstoffrest ersetzt sein kann.

5. Zusammensetzung nach Anspruch 1 oder 2, dadurch gekennzeichnet, daß das Silanol ein Siloxan vom Typ

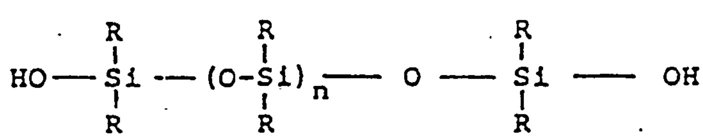

ist, worin R eine Kohlenwasserstoffgruppe ist und n eine Zahl von 0 bis 20, vorzugsweise von 0 bis 4.

6. Zusammensetzung nach einem der vorstehenden Ansprüche, dadurch gekennzeichnet, daß sie ein oder

mehrere Proteosilane

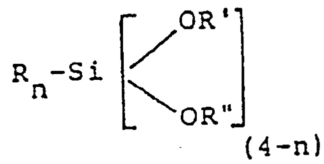

umfaßt, worin R eine Kohlenwasserstoffgruppe ist, insbesondere ein $C_1$- bis $C_{18}$-Alkyl oder -Alkenyl, n 1 bis 3 ist, R′ eine hautaktive Gruppe ist, insbesondere eine Theophyllin-, Theophyllinessigsäure-, Glycirrhizinsäure- oder Acetyltyrosingruppe und R″ eine Protidgruppe ist, insbesondere eine Lipoprotid-, Nukleoprotid-, Peptid-, Polypeptid oder Cyclopeptid-, besonders eine Albumin-, Fibrin-, Kollagen-, Keratin- oder Elastingruppe, wobei der Anteil an Proteosilan vorzugsweise 0,05 bis 0,5 Gew.-% beträgt.

7. Zusammensetzung nach einem der vorstehenden Ansprüche, dadurch gekennzeichnet, daß sie ein essentielles, insbesondere terpenisches Öl, vorzugsweise in einem Anteil von 0,05 bis 2 Gew.-% umfaßt.

8. Zusammensetzung nach einem der vorstehenden Ansprüche, dadurch gekennzeichnet, daß sie einen aktiven Pflanzenextrakt enthält, insbesondere aus Arnika, Honigklee, Ruscus, Ginkgo, Gemeinem Habichtskraut und/oder Centella asitica, vorzugsweise in einem Anteil von 0,05 bis 2 Gew.-%.

9. Zusammensetzung nach einem der vorstehenden Ansprüche, dadurch gekennzeichnet, daß sie in Form eines Gels, einer Emulsion oder einer Pomade vorliegt.

## Claims

1. Aqueous composition for the care of superficial lymphatic vessels, characterised in that it contains one or more silanols or silanol derivatives together with concholytic acid.

2. Composition according to claim 1, characterised in that it contains 0.05 to 0.5% by weight silanols or derivatives thereof, the amount of concholytic acid also being 0.05 to 0.5% by weight.

3. Composition according to claim 1 or 2, characterised in that the silanol(s) employed are of the type $R_nSi(OH)_{(4-n)}$, R being a hydrocarbon radical, particularly a $C_1$ to $C_{18}$ alkyl, and n = 1 to 3, one or more of the hydrogen atoms possibly being replaced by $C_1$ to $C_{18}$ alkyl radicals.

4. Composition according to claim 1 or 2, characterised in that the silanol derivatives are of the type

$$R_nSi(OH)_{(4-n)}.X.R'COOH$$

where R is a hydrocarbon group, particularly a $C_1$ to $C_{18}$ alkyl, n is 1 to 3, R′COOH being a pharmaceutically acceptable acid and X is (4-n), some of the hydrogen atoms of the $Si(OH)_{(4-n)}$ group possibly being replaced by hydrocarbon radicals.

5. Composition according to claim 1 or 2, characterised in that the silanol is a siloxane of the type

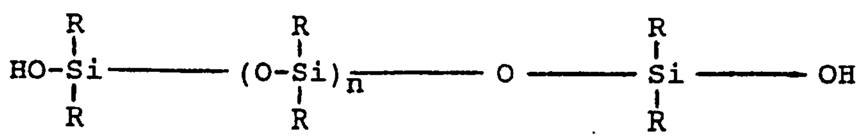

where R is a hydrocarbon group and n is a number from 0 to 20, preferably from 0 to 4.

6. Composition according to any of the preceding claims, characterised in that it contains one or more proteosilanes

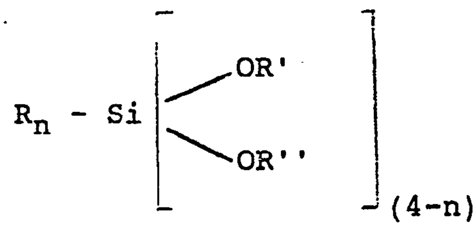

in which R is a hydrocarbon group, particularly $C_1$ to $C_{18}$ alkyl or alkenyl, n is 1 to 3, R′ is a group which is active to the skin, notably a theophylline group, theophylline acetic acid, glycyrrhizic acid or acetyltyrosine, and R″ is a protidic group, in particular lipoprotidic or nucleoprotidic, of peptides, polypeptides or cyclopeptides, notably albumin, fibrin, collagen, keratin or elastin, the proportion of proteosilane being preferably from 0.05 to 0.5% by weight.

7. Composition according to any of the preceding claims, characterised in that it contains an essential oil, notably terpenic, preferably in an amount from 0.05 to 2% by weight.

8. Composition according to any of the preceding claims, characterised in that it contains an active vegetable extract, particularly arnica, sweet clover, butcher's broom, ginkgo, mouse-ear hawkweed and/or centella asiatica, preferably in an amount of 0.05 to 2% by weight.

9. Composition according to any of the preceding claims, characterised in that it is in the form of a gel, an emulsion or a pomade.